# EUROPÄISCHE PATENTSCHRIFT

(11) **EP 1 446 076 B1**
(45) Veröffentlichungstag und Bekanntmachung des Hinweises auf die Patenterteilung: **06.12.2006**
(21) Anmeldenummer: 02803352.0
(22) Anmeldetag: 24.10.2002
(51) Int. Cl.: A61F 2/36

(54) **SCHENKELHALSENDOPROTHESE FÜR EIN KÜNSTLICHES HÜFTGELENK**
FEMORAL NECK ENDOPROSTHESIS FOR AN ARTIFICIAL HIP JOINT
ENDOPROTHESE DU COL DU FEMUR DESTINEE A UNE ARTICULATION DE HANCHE ARTIFICIELLE

(30) Priorität: 22.11.2001 DE 10158559
(43) Veröffentlichungstag der Anmeldung: 18.08.2004
(73) Patentinhaber: ESKA Implants GmbH & Co. KG, 23556 Lübeck (DE)
(72) Erfinder: GRUNDEI, Hans, 23558 Lübeck (DE); KARPF, Peter-Michael, 84028 Landshut (DE)
(74) Vertreter: Fuchs Mehler Weiss & Fritzsche
(86) Internationale Anmeldenummer: PCT/EP2002/011888
(87) Internationale Veröffentlichungsnummer: WO 2003/043543

(56) Entgegenhaltungen:
- EP-A- 0 094 829
- EP-A- 0 791 342
- EP-A- 1 205 163
- WO-A-89/11837
- DE-A- 2 724 234
- DE-A- 3 017 953
- DE-A- 3 334 058
- DE-C- 19 610 741
- DE-C- 19 720 493
- FR-A- 2 626 169
- FR-A- 2 674 122
- US-A- 2 765 787
- US-A- 5 376 126
- US-A- 5 755 793

## Beschreibung

Es wird eine Schenkelhalsendoprothese für ein künstliches Hüftgelenk beschrieben. Der nächstliegende Stand der Technik ist EP-A-0 791 342.

Zur Behandlung eines distruierten, d.h. zerstörten Hüftgelenkkopf, woraus erhebliche Schmerzempfindungen und gravierende funktionelle Einschränkungen des Gelenks resultieren, haben sich im wesentlichen zwei Therapiemöglichkeiten während der letzten Jahrzehnte durchgesetzt:

Zum einen ist es möglich, den Patienten mit einer Orthese zu versorgen, d.h. also mit einem externen Stützapparat. Neben der Tatsache, daß diese Versorgungsmöglichkeit kosmetisch völlig unakzeptable ist, ist sie auch versorgungstechnisch nur suboptimal.

Als weitere, weitverbreitete Möglichkeit ist es vorgesehen, den Patienten mit einer Endoprothese zu versorgen, namentlich mit einer Hüftstielendoprothese, bei welcher ein Stiel in den zuvor freigeräumten Knochenmarksraum des Femurs eingesetzt wird und dort zementlos oder unter Verwendung eines Zements fixiert wird. An der proximalen Seite weist eine derartige Endoprothese dann die Möglichkeit des Anschlusses einer künstlichen Gelenkkugel auf.

Gerade die letzte Möglichkeit ist stark in der Patentliteratur vertreten. An dieser Stelle sei nur beispielsweise auf die DE 94 12 408 U1 hingewiesen.

Unter den einen oder anderen der in der Patentliteratur vorgeschlagenen Hüftstielendoprothesen gibt es einige Ansätze, die recht vielversprechend sind im Hinblick auf die Langzeitstabilitiät im Patientenkörper. Früher oder später jedoch, beispielweise nach 10 bis 15 Jahren, ist zu beobachten, daß die Endoprothese einem Verschleiß unterworfen ist, welcher zu der Notwendigkeit führt, in einem Revisionseingriff die implantierte Endoprothese zu entfernen und durch eine neue zu ersetzen. Dies ist freilich nicht ganz unproblematisch, da durch die Resektion und Ausräumung des Knochenmarkraumes ein nicht unerheblicher Anteil natürlichen Knochenmaterials bei der Erstimplantation entfernt worden ist. Das einmal entfernte Material fehlt dann unter Umständen bei einem Revisionseingriff. Dies kommt insbesondere dann zum Tragen, wenn die Patienten relativ jung sind, bei denen also von vornherein davon auszugehen ist, daß sie im Laufe ihres Lebens mindestens einem Revisionseingriff unterworfen werden müssen.

Eine Endoprothese, die mit einer geringfügigeren Resektion natürlichen Knochenmaterials auskommt als die bekannten Hüftstielendoprothesen ist bekannt geworden aus der DE 27 24 234 C2. Das darin beschriebene Prothesenteil wird im oberen Bereich eines Femurs, aber unterhalb des Trochanter minors implantiert, ohne daß ein Stiel in den Knochenmarksraum reichen würde. Diese auch als Druckscheibenprothese bezeichnete Prothese greift im wesentlichen mit einer proximalen Druckplatte über die Kortikalis des resezierten Femurs im Bereich des entfernten Hüftgelenkkopfes. Sie wird mit einer Druckplatte, die lateral am Femur anliegt, verspannt, derart, daß alle mechanischen Kräfte zwischen der Endoprothese und dem Femur direkt in die Kortikalschicht des Femurs eingeleitet werden, wodurch eine als unzulässig empfundene mechanische Beanspruchung der Spongiosa vermieden werden soll. Lediglich eine geringe Menge von Knochenzement ist für die Arretierung der Druckscheibe im proximalen Bereich notwendig.

Der Philosophie dieser Prothese liegt - wie ausgeführt - die Annahme zugrunde, daß die Spongiosa des Femurs möglichst wenig beansprucht werden soll. Dies wird erkauft durch eine extreme Belastung der Kortikalis des Femurs, indem nämlich sämtliche Kräfte darauf abgelastet und darin eingeleitet werden. Dies entspricht nach heutiger Erkenntnis in keiner Weise den natürlichen Belastungsverhältnissen.

Eine ganz ähnlich wirkende Prothese zeigt im übrigen die WO 89/11837, bei der die Funktion der proximalen Druckplatte gemäß der vorerwähnten Druckschrift wahrgenommen wird durch den speziell ausgebildeten Hüftgelenkkopf, der innenseitig mit einer Ausnehmung versehen ist, an der sich innenseitig der reserzierte Femurstumpf unter Druckerzeugung anlegt.

Zwei Prothesen, bei denen augenscheinlich die Hauptlast von der Spongiosa des Femurknochens aufgenommen werden soll, sind bekannt aus der FR 26 26 169 A1 und FR 26 74 122 A1. Bei der erstgenannten Druckschrift der beiden ist vorgesehen, einen proximal einen Steckkonus aufweisenden Gewindebolzen in die Spongiosa einzuschrauben. Dies dürfte innerhalb kürzester Zeit zu schweren Instabilitätsproblem führen. Bei der zweitgenannten Druckschrift der beiden wird eine einen Steckkonus tragende Platte mittels einer Reihe von Knochenschrauben befestigt, wobei die Knochenschrauben in die Spongiosa reichen. Auch hieraus ergeben sich ernsthafte Stabilitätsfragen, da die Spongiosa von Natur aus im Verhältnis zur Kortikalis des Femurknochens punktuell nur gering belastbar ist.

Einen anderen Weg beschreitet die DE-A-196 01 340. In dieser Druckschrift wird eine Schenkelhalsendoprothese beschrieben, die eine zementlos im oberen Bereich eines Femurs unterhalb des Trochanter majors implantierbare Hülse aufweist, die an ihrem proximalen Ende mit einer künstlichen Gelenkkugel versehen ist. Ihre Hülsenaußenseite ist zumindest teilweise mit einer offenmaschigen dreidimensionalen Raumnetzstruktur belegt. Darüber hinaus ist eine Zugplatte vorgesehen, an der eine Zugschraube anschlagbar ist, die durch eine Durchbohrung im distalen Ende der Hülse in deren Inneren setzbar und mit einem dort versehenen Gewinde verschraubbar ist.

Zur Vorbereitung der Implantation wird zunächst der zerstörte Femurkopf durch eine subcapitale Resektion entfernt, und zwar im wesentlichen orthograd zur Schenkelhalsachse. Sodann wird mittels eines Fräsers, der eine im wesentlichen gleiche Außenkontur wie die zu implantierende Hülse aufweist, in den Schenkelhals getrieben und eine entsprechende Bohrung oder Aushöhlung in der Spongiosa des Femurs hergestellt. Sodann kann die Hülse in dem ausgefrästen Raum im Schenkelhals gesetzt werden. Zur lateralen Festlegung der Hülse ist vorgesehen, von außen an dem Femur die erwähnte Zugplatte anzulegen, welche mittels einer Zugschraube mit dem Inneren der Hülse verschraubbar ist. Zu diesem Zweck muß die Kortikalis lateral aufgebohrt werden, so daß die Zugschraube durch die Kortikalis in das Innere der Hülse geführt werden kann, um dort verschraubt zu werden. Die Zugplatte bildet mit der Zugschraube ein im Prinzip bekanntes System der Zuggurtung.

Im Gegensatz zu langstieligen Hüftlangstielendoprothesen findet die Endoprothese der vorerwähnten Art in der Metaphyse des Femurknochens Platz. Gegenüber den Langstielendoprothesen sind nur relativ geringe Resektionen von natürlichem Knochenmaterial notwendig, weswegen sie sich den natürlichen Verhältnissen erheblich besser anpaßt als jene der vorerwähnten Druckschriften. Hierzu trägt auch bei, daß die Hülse und das Hülsenende zumindest teilweise mit der erwähnten offenmaschigen dreidimensionalen Raumnetzstruktur belegt ist, in welche Knochentrapekel einwachsen und so für eine dauerhafte Fixation der Endoprothese in der Metaphyse sorgen.

Nun gibt es Indikationen, bei denen die Implantation der Endoprothese gemäß der vorerwähnten Druckschrift wenig erfolgsversprechend ist. Dies ist insbesondere dann der Fall, wenn die Einleitung von Belastungskräften in die Kortikalis durch die Zugschraube aufgrund von Schädigungen der Kortikalis problematisch ist. Gleichwohl besteht das Bedürfnis, auch die hiervon betroffenen Patienten mit einer Schenkelhalsendoprothese zu versorgen, um auch ihnen den Vorteil der geringstmöglichen Resektionen bieten zu können.

Vor diesem Hintergrund ist es nun die Aufgabe der vorliegenden Erfindung, eine Schenkelhalsendoprothese der vorerwähnten Art so weiterzubilden, daß sie in der Metaphyse des Femurs fixiert werden kann, ohne die Kortikalis zu durchbohren, welche zu Fehlfunktionen des Gelenkes führen können.

Gelöst wird diese Aufgabe durch eine Schenkelhalsendoprothese für ein künstliches Hüftgelenk mit den Merkmalen des Anspruchs 1. Weitere vorteilhafte Ausführungsformen sind in den Unteransprüchen angegeben.

Demgemäß weist die Schenkelhalsendoprothese auf:
- eine im oberen Bereich eines Femurs unterhalb des Trochanter majors implantierbare Hülse, deren Oberfläche zumindest teilweise mit einer dreidimensionalen offenmaschigen Raumnetzstruktur versehen ist und mit deren proximalem Ende ein Adapter in Form eines Koppelkonus zur Aufnahme einer Gelenkkugel verbindbar ist,
- eine Primärfixationshilfe am distalen Ende der Hülse in Form von auffächerbaren Arretierungsmitteln, die zur Anlage an die Innenseite der Kortikalis bestimmt sind, und
- ein Betätigungsmittel, mittels dessen die Arretierungsmittel von proximal aufgefächert werden.

Die erfindungsgemäße Schenkelhalsendoprothese wird also zementlos implantiert, wobei in bekannter Weise eine Seküundärfixation dadurch erzielt wird, daß Knochentrapekel in die offenmaschige Raumnetzstruktur einwächst und dort verknöchert. Die Endoprothese ist kragenlos, da insofern eine Ablastung ausschließlich in der Spongiosa der Metaphyse des Schenkelhalses stattfindet.

Darüber hinaus ist die erwähnte Primärfixationshilfe am distalen Ende der Hülse vorgesehen. Sie ist so ausgestaltet, daß sie die Kortikalis des Femurknochens nicht durchdringt, sondern sich vielmehr nur daran abstützt, um eine möglichst stabile Lage direkt nach der Implantation der Endoprothese zu erhalten. Nach Einwuchs von Knochenmaterial in die offenmaschige Raumnetzstruktur und nach Sicherstellung der Sekundärfixation erfüllt die Primärfixationshilfe im wesentlichen keine Funktion mehr.

Gemäß einer bevorzugten Ausführungsform sind die Arretierungsmittel aus lose in der Hülse gelagerten, an deren distalen Ende heraustretenden metallischen Drähten ausgebildet. Vorzugsweise sind mindestens fünf derartige Drähte vorgesehen, die sich dann in der lateralisierten Kortikalis aufspreizen lassen und so für einen guten Halt sorgen.

Das Betätigungsmittel ist besonders bevorzugt als ein Zylinderstift ausgebildet mit einem in eine Gewindebohrung im Inneren der Hülse schraubbaren Gewinde, an dessen verbreiterten Fuß die besagten Drähte anliegen. Dabei drückt der Fuß des Zylinderkopfes mit zunehmender Verschraubung mit der Gewindebohrung im Inneren der Hülse im zunehmenden Maße die Drähte aus der Hülse heraus. Die Handhabung ist also für den Operateur relativ einfach. Er muß einen Werkzeugkopf an den Zylinderstift heranführen und den Zylinderstift mit der Gewindebohrung im Inneren der Hülse verschrauben, wodurch dann die Drähte als Arretierungsmittel aus der Hülse herausgedrückt werden in die Spongiosa im Femurknochen hinein. Hierzu ist die Verteilung der Drähte vorzugsweise so vorzusehen, daß die Drähte in verschiedenen Raumrichtungen in die Spongiosa hineingreifen, um so den Bewegungsfreiheitsgrad der Hülse im Schenkelhals zu minimieren.

Die Lage der Schenkelhalsendoprothese in der Metaphyse des Femurs kann noch weiter dadurch stabilisiert werden, wenn die Hülse einen rotationsunsymmetrischen Querschnitt aufweist. Dies bedeutet, eine zusätzliche Sicherheit gegenüber einem Verdrehen der Endoprothese in der Metaphyse und damit eine Sicherheit gegen etwaige Fehlstellungen des künstlichen Hüftgelenkes.

Die Rotationsunsymmetrie kann bevorzugt dadurch realisiert werden, daß der Querschnitt der Hülse ovalär ist. In diesem Fall ist die Hauptachse des ovalären Hülsenquerschnitts besonders bevorzugt dorsal-ventral orientiert.

Alternativ kann ein Hülsenquerschnitt trapezförmig oder im wesentlichen dreieckig ausgebildet sein, wobei dann in vorteilhafter Weise die Ecken jeweils verrundet sind.

Die Erfindung wird nachfolgend anhand eines Ausführungsbeispieles näher erläutert. Hierbei zeigt
- Figur 1: die schematische Schnittansicht einer implantierten Schenkelhalsendoprothese im Schenkelhals eines Femurs, und
- Figur 2: schematisch eine Ansicht der Hülse in Richtung des Pfeiles II in Figur 1.

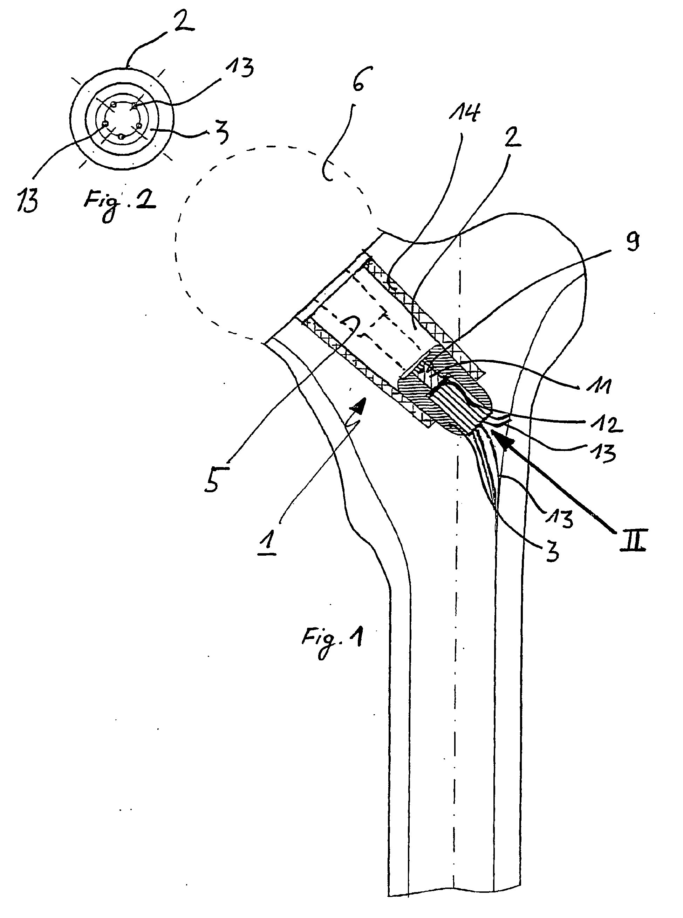

Figur 1 zeigt schematisch die implantierte Schenkelhalsendoprothese 1 in der Metaphyse des Femurknochens. Die Endoprothese 1 besteht aus einer Hülse 2, deren Oberfläche zumindest teilweise mit einer dreidimensionalen offenmaschigen Raumnetzstruktur 14 belegt ist. In diese wächst im Laufe der Zeit Knochenmaterial ein und verknöchert dort, so daß für eine langfristige Sekundärfixation gesorgt ist.

Für die Primärfixation ist eine Primärfixationshilfe am distalen Ende 3 der Hülse 2 vorgesehen. Diese weist auffächerbare Arretierungsmittel 13 in Form von metallischen Drähten auf, die lose in der Hülse 2 gelagert sind und aus deren distalen Ende 3 heraustreten. Die Drähte 13 sind flexibel. Darüber hinaus ist ein Betätigungsmittel 11 vorgesehen, welches einen Zylinderstift mit einem in eine Gewindebohrung 9 im Inneren der Hülse schraubbaren Gewinde aufweist. Der Zylinderstift 11 weist einen verbreiterten Fuß 12 auf, an welchem die Drähte 13 anliegen. Wird nun der Zylinderstift 11 zunehmend mit der Gewindebohrung 9 verschraubt, so drängt dessen verbreiterter Fuß 12 die an ihm anliegenden Drähte weiter aus der Hülse 2 an deren distalen Ende 3 hinaus. Da die Drähte 13 flexibel sind, suchen sie sich gewissermaßen in der Spongiosa ihren Weg, bis sie entweder an der Innenkortikalis des Femurs zu liegen kommen, oder aber in eine andere Raumrichtung abgebogen werden. Das Auffächern der Drähte in der Spongiosa sorgt für einen guten Halt der Endoprothese 1 im Schenkelhals des Femurs.

Gestrichelt angedeutet ist noch eine konische Klemmhülse 5 im Inneren der Hülse 2, mit der ein an sich bekannter Adapter in Form eines Doppelkonus verbindbar ist. Gleichfalls schematisch angedeutet ist eine künstliche Gelenkkugel 6.

### Bezugszeichen

- 1: Schenkelhalsendoprothese
- 2: Hülse
- 3: distales Ende
- 5: konische Steckhülse
- 6: künstliche Gelenkkugel
- 9: Gewindebohrung
- 11: Zylinderstift
- 12: verbreiterter Fuß v. 11
- 13: Draht
- 14: dreidimensionale offenmaschige Raumnetzstrukturen

## Patentansprüche

1. Schenkelhalsendoprothese, aufweisend
- eine im oberen Bereich eines Femurs unterhalb des Trochanter majors implantierbare Hülse (2), deren Oberfläche zumindest teilweise mit einer dreidimensionalen offenmaschigen Raumnetzstruktur (14) versehen ist und mit deren proximalem Ende ein Adapter in Form eines Doppelkonus zur Aufnahme einer Gelenkkugel (6) verbindbar ist, und
- eine Primärfixationshilfe am distalen Ende (3) der Hülse (2),
**dadurch gekennzeichnet, daß** die
Primärfixationshilfe in Form von auffächerbaren Arretierungsmitteln (13) ist, die zur Anlage an die Innenseite der Kortikalis bestimmt sind, und daß die Schenkelhalsendoprothese
ein Betätigungsmittel (11) aufweist, mittels dessen die Arretierungsmittel (13) von proximal aufgefächert werden.

2. Schenkelhalsendoprothese nach Anspruch 1, bei der die Arretierungsmittel (13) gebildet sind aus lose in der Hülse (2) gelagerten, an deren distalen Ende (3) heraustretenden metallischen Drähten.

3. Schenkelhalsendoprothese nach Anspruch 2, bei der das Betätigungsmittel (11) einen Zylinderstift mit einem in eine Gewindebohrung (9) in Inneren der Hülse (2) schraubbaren Gewinde aufweist, an dessen verbreitertem Fuß (12) die Drähte (13) anliegen, wobei der Fuß (12) des Zylinderstiftes (11) mit zunehmender Verschraubung in zunehmendem Maße aus der Hülse (2) die Drähte (13) herausdrückt.

4. Schenkelhalsendoprothese nach Anspruch 2 oder 3, bei der wenigstens fünf Drähte (13) als Arretierungsmittel vorgesehen sind.

5. Schenkelhalsendoprothese nach einem der Ansprüche 1 bis 4, bei der die Hülse (2) einen rotationssymmetrischen Querschnitt aufweist.

6. Schenkelhalsendoprothese nach Anspruch 5, bei der der Querschnitt der Hülse (2) ovalär ist.

7. Schenkelhalsendoprothese nach Anspruch 6, bei der die Hauptachse des ovalären Hülsenquerschnitts dorsal-ventral orientiert ist.

8. Schenkelhalsendoprothese nach Anspruch 5, bei der der Querschnitt der Hülse (2) trapezförmig ist.

9. Schenkelhalsendoprothese nach Anspruch 5, bei der der Querschnitt der Hülse (2) im wesentlichen dreieckig ist.

10. Schenkelhalsendoprothese nach Anspruch 8 oder 9, bei der die Ecken des Querschnitt verrundet sind.

## Claims

1. Femoral neck endoprosthesis having
- a sleeve (2) which can be implanted in the upper region of a femur below the trochanter major, the surface of the sleeve (2) being provided at least partly with a three-dimensional open-mesh spatial network structure (14) and an adapter in the form of a double cone can be connected to its proximal end to receive a joint head (6), and
- a primary fixing aid on the distal end (3) of the sleeve (2),
**characterised in that** the primary fixing aid is in the form of locking means (13) which can be fanned out and which are intended to rest on the inner side of the cortex, and **in that** the femoral neck endoprosthesis has an actuating means (11), by means of which the locking means (13) are fanned out proximally.

2. Femoral neck endoprosthesis according to claim 1, in which the locking means (13) are formed from metallic wires loosely mounted in the sleeve (2) and emerging at the distal end (3) thereof.

3. Femoral neck endoprosthesis according to claim 2, in which the actuating means (11) has a cylindrical pin with a thread which can be screwed into a threaded bore (9) in the interior of the sleeve (2), on the widened base (12) of which thread the wires (13) rest, wherein the base (12) of the cylindrical pin (11) presses the wires (13) out of the sleeve (2) to an increasing extent with increasing screwing.

4. Femoral neck endoprosthesis according to claim 2 or 3, in which at least five wires (13) are provided as locking means.

5. Femoral neck endoprosthesis according to one of claims 1 to 4, in which the sleeve (2) has a rotationally symmetrical cross-section.

6. Femoral neck endoprosthesis according to claim 5, in which the cross-section of the sleeve (2) is oval.

7. Femoral neck endoprosthesis according to claim 6, in which the main axis of the oval sleeve cross-section is orientated dorsally-ventrally.

8. Femoral neck endoprosthesis according to claim 5, in which the cross-section of the sleeve (2) is trapezoidal.

9. Femoral neck endoprosthesis according to claim 5, in which the cross-section of the sleeve (2) is essentially triangular.

10. Femoral neck endoprosthesis according to claim 8 or 9, in which the corners of the cross-section are rounded.

## Revendications

1. Endoprothèse du col du fémur présentant un manchon (2) pouvant être implanté dans la zone supérieure d'un fémur en dessous du grand trochanter, dont la surface est au moins munie d'une structure (14) à mailles ouvertes tridimensionnelle à l'extrémité proximale de laquelle un adaptateur sous forme d'un double cône peut être relié pour recevoir une rotule d'articulation (6), et un auxiliaire de fixation primaire sur l'extrémité distale (3) du manchon (2),
**caractérisée en ce que** l'auxiliaire de fixation primaire est en forme de moyens de blocage (13) pouvant être déployés qui sont destinés à reposer sur le côté interne de la corticale, et **en ce que** l'endoprothèse présente un moyen d'actionnement (11) au moyen duquel les moyens de blocage (13) peuvent être déployés depuis la proximale.

2. Endoprothèse du col du fémur selon la revendication 1, dans laquelle les moyens de blocage (13) sont formés de fils métalliques placés de manière libre dans le manchon (2) faisant saillie sur son extrémité distale (3).

3. Endoprothèse du col du fémur selon la revendication 2, dans laquelle le moyen d'actionnement (11) présente une tige de cylindre avec un filetage pouvant être vissé dans un perçage fileté (9) à l'intérieur du manchon (2), les fils (13) reposant sur sa base élargie (12), la base (12) de la tige de cylindre (11) pressant les fils (13) hors du manchon (2) dans une mesure croissante avec un vissage croissant.

4. Endoprothèse du col du fémur selon la revendication 2 ou 3, dans laquelle au moins cinq fils (13) sont prévus comme moyens de blocage.

5. Endoprothèse du col du fémur selon l'une des revendications 1 à 4, dans laquelle le manchon (2) présente une section symétrique en rotation.

6. Endoprothèse du col du fémur selon la revendication 5, dans laquelle la section du manchon (2) est ovale.

7. Endoprothèse du col du fémur selon la revendication 6, dans laquelle l'axe principal de la section ovale de manchon est orienté de manière dorsale-ventrale.

8. Endoprothèse du col du fémur selon la revendication 5, dans laquelle la section du manchon (2) trapézoïdale.

9. Endoprothèse du col du fémur selon la revendication 5, dans laquelle la section du manchon (2) est globalement triangulaire.

10. Endoprothèse du col du fémur selon la revendication 8 ou 9, dans laquelle les angles de la section transversale sont arrondis.
